(19) Europäisches Patentamt
European Patent Office
Office européen des brevets

(11) EP 0 694 876 B1

(12) FASCICULE DE BREVET EUROPEEN

(45) Date de publication et mention de la délivrance du brevet:
**24.05.2000 Bulletin 2000/21**

(51) Int. Cl.[7]: **G06T 7/00**

(21) Numéro de dépôt: **94490034.9**

(22) Date de dépôt: **29.07.1994**

# (54) Procédé d'acquisition et de traitement de l'image d'un article plan, du type étoffe de tissu, en vue de la détection de défauts de fabrication

Verfahren zur Gewinnung und Verarbeitung des Bilds eines flachen Artikels sowie Gewebestoff für die Feststellung von Herstellungsdefekten

Tissue material type flat article acquisition and processing method in order to detect manufacturing defects

(84) Etats contractants désignés:
**DE GB IT SE**

(43) Date de publication de la demande:
**31.01.1996 Bulletin 1996/05**

(73) Titulaire:
**Centre Technique Industriel dit:
INSTITUT TEXTILE DE FRANCE
92223 Bagneux Cédex (FR)**

(72) Inventeur: **Guermonprez, Philippe
F-59700 Marcq en Baroeul (FR)**

(74) Mandataire:
**Hénnion, Jean-Claude et al
Cabinet Beau de Loménie,
37, rue du Vieux Faubourg
59800 Lille (FR)**

(56) Documents cités:
- **PROCEEDINGS OF THE 1988 IEEE INTERNATIONAL CONFERENCE ON SYSSTEMS,MAN, AND CYBERNETICS, AUGUST 8-12,1988 ,BEIJING AND SHENYANG, CHINA ;IAP, XP92243 JIAHAN CHEN ET AL. 'A structural approach to identify defects in textured images'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 694 876 B1

## Description

La présente invention a pour objet un procédé d'acquisition et traitement de l'image d'un article plan à structure périodique, du type étoffe de textile tissée, en vue de la détection de défauts de fabrication dans cet article.

Dans le domaine du tissage, chaque pièce de tissu peut se caractériser par une armure plus ou moins complexe correspondant à un motif élémentaire qui se répète périodiquement dans tout le tissu, en fonction du mode d'entrecroisement des fils de chaîne et de trame. La structure périodique du tissu est donc définie par la taille de l'armure et par les deux directions dans lesquelles l'armure se répète. Ces deux directions correspondent approximativement aux directions des fils de trame et de chaîne constituant les deux voies naturelles de formation du tissu.

Lorsqu'un défaut apparaît au moment de la fabrication du tissu, celui-ci se traduit par un défaut de périodicité dans la structure du tissu. Dans la pratique, la détection des éventuels défauts de fabrication est effectuée, une fois que le tissu est déjà entièrement terminé , lors d'une opération complémentaire appelée visitage. Cette opération consiste à dérouler la pièce de tissu sur une table de visite devant un opérateur qui contrôle visuellement la qualité de la pièce ; l'opérateur arrête le déroulement de la pièce lorsqu'il voit un défaut et positionne en lisière une sonnette de repérage du défaut.

L'opération de visitage telle que décrite ci-dessus reste cependant une source d'erreurs et ne permet pas des cadences de traitement importantes.

En vue de pallier ces inconvénients, on a déjà proposé, notamment par le document US 4 619 527, de remplacer le visitage humain, par une détection automatique des défauts de fabrication dans la pièce de tissu, qui est basée sur l'acquisition d'une image initiale numérique du tissu, et sur le traitement de cette image. La pièce de tissu est déroulée en continu et à plat sur une table de visite, en étant éclairée par transmission et/ou réflexion par un flux de lumière. Un dispositif d'acquisition d'images, utilisant des capteurs optoélectriques qui transforment la lumière transmise et/ou réflective par les fibres du tissu en un signal analogique qui est ensuite numérisé, acquiert une image numérique de la surface du tissu, laquelle image est filtrée en vue de supprimer la texture générale du tissu dans l'image. Cette texture générale est liée à l'entrecroisement régulier des fils de trame et de chaine du tissu. Le principe qui est à la base du filtrage de l'image consiste à effectuer la différence entre deux valeurs de luminosité qui sont obtenues en effectuant une moyenne de la luminosité traversant deux fentes identiques, et parallèles à la direction soit des fils de trame soit des fils de chaine.

Le procédé de filtrage décrit dans le document US 4,619,597 peut être mis en oeuvre soit à l'aide de filtres optiques appropriés, soit en effectuant un traitement électronique sur l'image initiale qui a été acquise de la surface du tissu. L'inconvénient de ce procédé de filtrage réside dans l'absence de prise en compte de la périodicité de la structure du tissu.

Afin de tenir compte de cette périodicité, il a déjà été proposé , notamment dans la demande de brevet européen EP.243639, de filtrer l'image initiale acquise, dans un premier temps en affectant à chaque point élémentaire un niveau de gris résultant de la comparaison du niveau de gris dudit point élémentaire avec le niveau de gris du point élémentaire qui lui est voisin à une période près, et dans un deuxième temps en rendant binaire ce niveau de gris par comparaison avec un seuil prédéterminé.

Le problème qui se pose dans ce cas est la détermination de ce seuil de filtrage. En effet, il s'avère qu'un seuil de filtrage d'une valeur donnée ne procure pas le même résultat, au niveau de la qualité du filtrage, avec deux tissus distincts.

Le but que s'est fixé le demandeur est de proposer un procédé d'acquisition et de traitement de l'image d'un article plan à structure périodique, qui pallie l'inconvénient constaté en ce qu'il tient compte de la spécificité de chaque article , et permet le calcul d'un seuil de filtrage adapté à chaque article.

Ce but est parfaitement atteint par le procédé de l'invention , qui de manière connue, notamment par la demande de brevet européen EP.243639 , consiste :

a) à acquérir une image initiale de la surface de l'article, laquelle image se caractérise au moins par un vecteur période $\vec{t}$ (respectivement $\vec{c}$),

b) et simultanément ou successivement à filtrer l'image initiale en vue d'obtenir une image filtrée en noir et blanc,

- b1) en calculant pour chaque point élément P de l'image initiale, un niveau de gris NG'(P) qui est proportionnel à la valeur absolue de la différence des niveaux de gris dans l'image initiale du point élémentaire P et de son homologue A (respectivement D) par la translation de vecteur période $\vec{t}$ (respectivement $\vec{c}$),
- b2) en affectant au point élémentaire P, le niveau de gris calculé à l'étape précédente,
- b3) et en rendant binaire le niveau de gris de chaque point élémentaire P par comparaison avec un premier seuil $S_{1t}$ (respectivement $S_{1c}$).

De manière caractéristique selon l'invention, le procédé comprend une étape préalable , dite d'apprentissage, qui comporte la succession d'étapes suivantes :

- acquérir une image numérique dite d'apprentissage d'une partie de la surface de l'article qui est dépourvue de défauts,
- appliquer les étapes b1) à b2) précitées sur l'image d'apprentissage,
- calculer l'histogramme des niveaux de gris de l'image d'apprentissage filtrée de vecteur période $\vec{t}$ (respectivement $\vec{c}$),
- calculer automatiquement le premier seuil $S_{1t}$ (respectivement $S_{1c}$) à partir de l'histogramme des niveaux de gris de l'image d'apprentissage filtrée.

Il était certes connu, notamment par la demande de brevet européen EP.496086 , d'acquérir une image d'apprentissage de la partie d'un tissu dépourvu de défauts, et d'utiliser cette image d'apprentissage pour déterminer les vecteurs trame ou chaîne du tissu. Néanmoins , ce document n'enseignait pas le calcul d'un seuil de filtrage à partir de l'histogramme de cette image d'apprentissage, filtrée conformément à l'enseignement de la demande de brevet européen EP.0243639.

Il convient de noter que le filtrage de l'image initiale correspondant à l'étape b) précitée, peut être éffectué une fois que l'image a été acquise et mémorisée. Il peut également être avantageusement réalisé en temps réel en cours d'acquisition de l'image initiale.

De préférence, pour effectuer le calcul du premier seuil $S_{1t}$ ou $S_{1c}$ on calcule préalablement la fonction de répartition F(S) des niveaux de gris de l'histogramme de l'image d'apprentissage filtrée , puis on détermine automatiquement le premier seuil comme étant le plus petit seuil S tel que F (S) est supérieur ou égal à (1 - ε), ε étant un coefficient prédéterminé, inférieur à 1. Plus particulièrement, dans le but d'améliorer l'image filtrée en noir et blanc obtenue à l'issue de l'étape b), le procédé de l'invention comprend avantageusement une étape supplémentaire c) de filtrage consistant à transformer la deuxième image numérique en noir et blanc obtenue à l'étape b3), d'une part en calculant pour chaque point élémentaire P de ladite deuxième image, la somme des niveaux de gris des points élémentaires situés dans une fenêtre élémentaire qui est de forme et de dimension prédéterminées, et qui contient le point élémentaire P, et d'autre part en affectant la valeur binaire 0 ou 1 au niveau de gris de chaque point élémentaire P, en fonction du résultat de la comparaison de ladite somme avec un deuxième seuil $S_{2t}$ (respectivement $S_{2c}$) prédéterminé.

La forme et la dimension de la fenêtre élémentaire dépendent du type de défaut que l'on souhaite détecter. En pratique, s'agissant de fenêtres élémentaires de forme rectangulaire, la largeur du rectangle sera fonction de la finesse des défauts que l'on souhaite détecter, et la longueur du rectangle sera fonction de la longueur de ces défauts.

De préférence, la fenêtre élémentaire contenant n points élémentaires, le deuxième seuil $S_{2t}$ (respectivement $S_{2c}$) est obtenu en calculant l'expression suivante :

$$3\sqrt{F(S_{1t})(1-F(S_{1t}))n} + n(1-F(S_{1t}))$$

Le deuxième seuil $S_{2t}$ est donc fonction du premier seuil $S_{1t}$.

Le vecteur période $\vec{t}$ ou $\vec{c}$ peut être déterminé à partir de la période réelle de la structure de l'article, et de la résolution du dispositif permettant l'acquisition de l'image initiale. Le vecteur période $\vec{t}$ ou $\vec{c}$ peut également être extrapolé à partir de la valeur des niveaux de gris de l'image d'apprentissage.

D'autres caractéristiques et avantages de l'invention ressortiront de la description suivante de variantes particulières de réalisation du procédé de l'invention, appliquées à la détection de défauts de fabrication dans une étoffe de tissu, donnée en référence au dessin annexé sur lequel :

La figure 1 est une représentation schématique générale d'un dispositif particulier permettant la mise en oeuvre du procédé de l'invention,

La figure 2 est un schéma bloc des moyens électroniques du dispositif de la figure 1,

Les figures 3A et 3B sont des schémas blocs de deux modes particuliers de réalisation des moyens de filtrage qui sont utilisés dans les moyens de traitement électronique de la figure 2, et qui permettent la mise en oeuvre des étapes b1) à b3) du procédé de l'invention,

La figure 4 est une représentation schématique de l'image initiale qui est acquise de la surface de l'étoffe de tissu,

La figure 5 est une représentation schématique d'une partie de l'image en noir et blanc obtenue à l'étape b3) et faisant apparaître une fenêtre élémentaire particulière pour la réalisation de l'étape c),

La figure 6A représente le signal de luminance qui est délivré par la barrette de photodiodes utilisée dans le dispositif de la figure 1,

Les figures 6B et 6C sont des courbes permettant d'illustrer le phénomène d'écho à partir du signal de luminance de la figure 6A,

La figure 7A illustre une image initiale particulière,

Les figures 7B à 7F illustrent respectivement les images numériques obtenues à partir du filtrage de l'image initiale de la figure 7A,

La figure 7G correspond à l'image de la figure 7A, sur laquelle apparaissent les limites des défauts qui ont été détectés par le procédé de l'invention,

La figure 8 est une représentation schématique de l'image d'apprentissage, sur laquelle sont représentés certains vecteurs prédéterminés,

Les figures 9A et 9B représentent des organigrammes de fonctionnement permettant le calcul des vecteurs période de l'image initiale de l'étoffe de tissu, et

La figure 9C est un organigrammme illustrant les différentes étapes permettant le calcul des premier et deuxième seuils, pour un vecteur période donné.

Le procédé d'acquisition et de traitement d'image de l'invention permet notamment la détection de défauts de fabrication dans une étoffe 1 de tissu, telle que celle représentée à la figure 1. Il consiste à acquérir une image initiale numérique de la surface de l'étoffe (étape a)) et simultanément ou successivement à cette acquisition, à filtrer l'image initiale numérique obtenue (étape b)). L'acquisition de l'image initiale numérique peut être faite sous un éclairage cohérent ou non cohérent.

Selon un procédé d'acquisition d'image connu de l'homme du métier, et donné à titre d'exemple non limitatif, l'étape a) d'acquisition est réalisée en faisant défiler l'étoffe 1 en continu, dans le sens de la flèche D, à l'aide d'un dispositif enrouleur-dérouleur 2a, 2b, devant une caméra ligne type CCD. La prise d'images effectuée par cette caméra est synchronisée avec la vitesse de défilement de l'étoffe 1.

La caméra ligne qui est représentée schématiquement à la figure 1 consiste d'une part en une barrette 3 de photodiodes, qui permet l'acquisition à chaque prise d'image d'une ligne 1a de l'étoffe 1, en transformant la lumière réfléchie et transmise par l'étoffe, en un signal analogique 8 dit de luminance, et d'autre part en un convertisseur analogique-numérique 4, qui code de façon synchrone avec le signal d'horloge 10, le signal de luminance 8 en un premier signal numérique 9 sur huit bits, dont les valeurs instantanées successives constituent les niveaux de gris des points élémentaires de l'image initiale numérique de l'étoffe 1. Ce premier signal numérique 9 est transformé par l'intermédiaire de moyens électroniques 5 en un deuxième signal numérique 11, dont les valeurs instantanées successives constituent les niveaux de gris des points élémentaires d'une deuxième image numérique, qui peut être affichée pour un opérateur à l'aide de moyens de visualisation 6, qui sont par ailleurs déjà connus.

On a représenté à la figure 7A l'image initiale de l'étoffe de tissu 1, qui serait affichée par les moyens de visualisation 6, si ceux-ci étaient reliés au premier signal numérique 9. Une vue partielle de cette image initiale a également été représentée de façon schématique à la figure 4. Cette image est constituée de lignes 29a qui sont composées de points élémentaires 29b, encore appelés pixels.Chaque ligne 29a correspond à une prise d'image par la caméra ligne. Les directions X et Y correspondent respectivement aux directions générales des fils de trame et de chaîne de l'étoffe de tissu 1. Il apparaît donc clairement à la figure 4 que la barrette 3 de photodiodes a été orientée en sorte d'être parallèle à la direction des fils de trame. Cette orientation particulière, mais qui n'est pas nécessaire à la réalisation du procédé d'acquisition et de traitement de l'invention, a été choisie dans un souci de simplification de l'exposé de ce procédé.

L'image initiale numérique représentée à la figure 4 se caractérise par deux vecteurs période $\vec{t}$ et $\vec{c}$ qui ont pour axe principal respectivement l'axe X et Y. Ces deux vecteurs période $\vec{t}$ et $\vec{c}$ de l'image initiale numérique correspondent à la double périodicité de l'armure de l'étoffe de tissu 1.

Si l'on considère un point élémentaire particulier P de l'image initiale numérique, celui-ci a pour homologues les points élémentaires A et D respectivement par les translations de vecteur $\vec{t}$ et $\vec{c}$. De même, ce point élémentaire P est l'homologue des points élémentaires C et B respectivement par les translations de vecteur $\vec{t}$ et $\vec{c}$. Le principe sur lequel repose le filtrage de l'étape b) du procédé de l'invention consiste, pour chaque vecteur période $\vec{t}$ et $\vec{c}$, a transformer l'image initiale numérique qui a été acquise, en une deuxième image numérique, en calculant pour chaque point élémentaire P de l'image initiale un nouveau niveau de gris, à partir des niveaux de gris du point élémentaire P et des niveaux de gris des points élémentaires qui lui sont voisins à une période près, à savoir les points élémentaires A et C ou B et D selon qu'il s'agit du vecteur période $\vec{t}$ ou $\vec{c}$.

Les moyens de traitement électronique 5, dont l'architecture et le fonctionnement vont être à présent décrits en référence aux figures 2, 3A et 3B, permettent la mise en oeuvre de l'étape b) de filtrage de l'invention. Ces moyens de traitement électronique 5 comportent en entrée quatre registres à décalage 13a, 13b, 13c et 13d qui sont connectés dans cet ordre en cascade, le registre 13a recevant en entrée le premier signal numérique 9. La taille de ces registres est programmée en sorte que les registres 13a et 13d puissent contenir n octets, et les registres 13b et 13c puissent contenir m octets. Ces registres sont en outre tous synchronisés par le même signal d'horloge H, qui est constitué par le signal d'horloge 10 du convertisseur analogique-numérique 4. Ainsi, les octets qui sont générés par le convertisseur analogique-numérique, et qui correspondent au niveau de gris des points élémentaires successifs de l'image initiale numérique, progressent à chaque impulsion du signal d'horloge H, dans la file de données constituée par les registres

13a, 13b, 13c et 13d.

Sachant que chaque ligne 29a de l'image initiale comporte n' points élémentaires 29b, et que les vecteurs $\vec{t}$ et $\vec{c}$ ont pour coordonnées respectives (tx, ty) et (cx, cy), les nombres d'octets contenus dans les registres à décalage sont donnés par les deux relations suivantes :

$$m = ty.n' + tx$$

$$n = cy.n' + cx - m$$

Dans l'exemple illustré à la figure 4, les vecteurs $\vec{t}$ et $\vec{c}$ ont pour coordonnées respectives (4,1) et (2,3). Dans ce cas, les registres 13a et 13d sont programmés pour contenir 2046 octets et les registres 13b et 13d sont programmés pour contenir 1028 octets. De la sorte si l'on considère, à un instant donné, que le signal numérique 14 reliant les registres 13b et 13c, représente le point élémentaire P de la figure 4, il en résulte que les signaux numériques 15 et 16 correspondent respectivement aux points élémentaires A et C. De la même façon, le signal numérique 9 et le signal numérique 12 qui est délivré en sortie par le registre en décalage 13d correspondent respectivement aux points élémentaires D et B.

Les moyens de traitement électronique 5 comportent également des premiers 17 et deuxièmes 26 moyens de filtrage permettant de traiter en parallèle respectivement les trois signaux 14, 15 et 16 et les trois signaux 14, 9 et 12.

Un exemple de réalisation des premiers moyens de filtrage 17 est détaillé à la figure 3A. Ceux-ci comportent en entrée des premiers 18a et 19a, et deuxièmes 18b et 19b, moyens de comparaison qui délivrent respectivement en sortie des signaux 20a et 20b qui correspondent respectivement à la valeur absolue de la différence des deux signaux 14 et 15 et à la valeur absolue de la différence des deux signaux 14 et 16. Les signaux 20a et 20b sont ensuite transformés par des troisièmes moyens de comparaison 21 en un signal 21a ayant pour valeur la plus petite valeur des deux signaux 20a et 20b. Le signal 21a est ensuite transformé par des moyens de seuillage 22, en un signal binaire 23 valant 0 ou 1 selon que la valeur du signal 21a est strictement inférieure ou supérieure à un premier seuil prédéterminé $S_{1t}$.

En connectant directement les moyens de visualisation 6 à la sortie des premiers moyens de comparaison 18a, 19a, on met en oeuvre le procédé de traitement de l'invention selon une première variante de réalisation qui consiste à appliquer à chaque point élémentaire P de l'image initiale, dont les niveaux de gris sont générés par le convertisseur analogique-numérique 4, la succession d'étapes suivantes :

étape b1) calculer un niveau de gris NG'(P) qui correspond à la valeur instantanée du signal 20a et qui est égal à la valeur absolue de la différence des niveaux de gris des points élémentaires P et A;
étape b2) affecter au point élémentaire P le niveau de gris NG'(P) qui a été calculé à l'étape précédente.

La figure 7B représente la deuxième image filtrée que l'on obtient à partir de l'image initiale de la figure 7A, lorsque l'on connecte les moyens de visualisation 6 au signal 20a, pour la mise en oeuvre de cette première variante de réalisation.

Si l'on connecte directement les moyens de visualisation 6 à la sortie des troisièmes moyens de comparaison 21, on met en oeuvre le procédé de traitement de l'invention selon une deuxième variante de réalisation qui consiste d'une part à effectuer à l'étape b1) précitée le calcul d'un niveau de gris NG"(P) qui correspond à la valeur instantanée du signal 20b, et qui est égal à la valeur absolue de la différence des niveaux de gris des points élémentaires P et C, et d'autre part à effectuer préalablement à l'étape b2) précitée une étape supplémentaire b'1) consistant à déterminer un niveau de gris NG(P) qui correspond à la valeur instantanée du signal 21a et qui est égal à la plus petite valeur des niveaux de gris NG'(P) et NG"(P). La figure 7C illustre la deuxième image initiale filtrée qui est obtenue à partir de la première image initiale de la figure 7A, dans le cadre de cette deuxième variante de réalisation.

Si l'on connecte directement les moyens de visualisation 6 à la sortie des moyens de seuillage 22, on met en oeuvre le procédé de traitement de l'invention selon une troisième variante de réalisation dans laquelle on effectue après l'étape b2 une étape supplémentaire b3 qui consiste à rendre binaire le niveau de gris NG(P) qui a été calculé à l'étape b2, en le comparant à un premier seuil prédéterminé $S_{1t}$. L'image en noir et blanc qui est dans ce cas obtenue est illustrée à la figure 7D. Sur cette figure, les points élémentaires susceptibles de représenter un défaut apparaissent en blanc, et correspondent à tous les points élémentaires dont le niveau de gris NG(P) était supérieur au premier seuil $S_{1t}$.

Sur l'image initiale de la figure 7A représentant la surface de l'étoffe 1, apparaît très nettement la texture générale de cette étoffe, qui correspond à l'impression visuelle rendue par l'entrecroisement des fils de trame et de chaîne du tissu. Dans cette image initiale, apparaît déjà une irrégularité dans cette texture générale, qui est due à un fil manquant en chaîne. Sur l'image filtrée de la figure 7B, la texture générale du tissu a été supprimée et l'irrégularité correspondant au fil manquant en chaîne apparaît plus nettement. Sur la figure 7C le contour de cette irrégularité est plus fin, car on a supprimé le phénomène d'écho qui va être à présent détaillé en référence aux figures 6A, 6B et 6C.

On a représenté à la figure 6A le signal de luminance 8 susceptible d'être obtenu par exemple en branchant un oscilloscope à la sortie de la barrette 3 de photodiodes. Dans un souci de simplification, on considère que la structure de l'étoffe est périodique dans une direction qui est confondue avec la direction X des fils de chaîne. Ainsi, le signal de luminance de la figure 6A est périodique et a pour période p. Un des pics 32a de ce signal traduit une irrégularité dans la périodicité du signal de luminance car son intensité est supérieure à celle de ses deux voisins 32b et 32c à une période p près. Si l'on affecte à chaque point de la courbe de la figure 6A une intensité lumineuse correspondant à la valeur absolue de la différence de l'intensité lumineuse de ce point et de l'intensité lumineuse du point qui lui est voisin à une période près dans le sens croissant de la direction X, on obtient la courbe en pointillés de la figure 6B qui est composée de deux pics 33b et 33a. De la même manière, si l'on affecte à chaque point de la figure 6A, une intensité lumineuse correspondant à la valeur absolue de la différence de l'intensité lumineuse de ce point avec l'intensité lumineuse du point qui lui est voisin à une période près dans le sens décroissant de la direction X, on obtient la courbe en trait plein de la figure 6B, qui se caractérise par deux pics 34a et 34c. Dans les deux cas, à partir d'un seul pic 32a représentant un unique défaut dans la périodicité de la structure, on génère deux pics 33b et 33a, ou 34a et 34c. C'est ce que l'on appelle communément le phénomène d'écho. Si pour chaque point de même abscisse on ne garde que la plus petite des deux valeurs des deux courbes représentées à la figure 6B, on obtient la courbe de la figure 6C qui se traduit par un unique pic 35 ayant la même abscisse que le pic 32a. On a donc réussi à supprimer ce phénomène d'écho. Ce même principe de suppression d'écho s'applique aux niveaux de gris des points élémentaires de l'image initiale qui résultent d'une numérisation du signal de luminance 8.

On a représenté à la figure 3B, un deuxième exemple de réalisation des premiers moyens de filtrage 17 qui sont plus faciles techniquement à réaliser, mais qui ne permettent pas d'obtenir l'image de la figure 7C.

Parmi les points élémentaires de l'image filtrée de la figure 7D, seuls les points élémentaires qui apparaissent en blanc, et qui sont suffisamment regroupés, caractérisent un défaut réel de fabrication dans l'étoffe 1. Afin d'éliminer tous les points élémentaires blancs et isolés, on effectue après l'étape b3) une dernière étape c dite de validation. Cette étape de validation, qui est par ailleurs déjà connue de l'homme du métier, consiste par exemple, si l'on se réfère à la figure 5, pour chaque point élémentaire P de l'image 30 obtenue à l'étape b3, à effectuer la somme des niveaux de gris des points élémentaires situés dans une fenêtre 31 contenant le point élémentaire P, à comparer cette somme avec un deuxième seuil prédéterminé $S_{2t}$, et à affecter la valeur binaire 0 ou 1 au point élémentaire P en fonction du résultat de cette comparaison. Cette étape peut être mise en oeuvre par des premiers moyens de validation 24, par ailleurs déjà connus de l'homme du métier, qui transforment le signal numérique 23 en un signal numérique 25 à destination des moyens de visualisation 6. La forme et la dimension de la fenêtre 31 dépendent de la taille et de la forme des défauts que l'on souhaite détecter. En l'occurrence, cette fenêtre est un rectangle dont la longueur L coïncide avec la direction des fils de chaîne et la largeur l coïncide avec la direction des fils de trame. Les premiers moyens de filtrage 17 et les premiers moyens de validation 24 constituent une voie de traitement appelée voie chaîne, qui permet la détection des défauts qui ne sont pas colinéaires au vecteur $\vec{t}$, et plus particulièrement des défauts de chaîne. On peut donc raisonnablement s'attendre à ce que les défauts que l'on cherche à détecter aient une forme allongée dans le sens des fils de chaîne, ce qui justifie le choix de la fenêtre rectangulaire 31.

Les moyens de traitement électronique 5 comportent également une voie de traitement dite voie trame, qui permet la détection notamment des défauts de trame et qui est constituée par les deuxièmes moyens de filtrage 26 et par des deuxièmes moyens de validation 27. Dans ce cas, les fenêtres de validation des défauts seront de préférence des rectangles dont la longueur coïncide avec la direction des fils de trame. Les deuxièmes moyens de filtrage 26 et les deuxièmes moyens de validation 27 sont similaires respectivement aux premiers moyens de filtrage 17 et aux premiers moyens de validation 24 et ne s'en différencient que par la valeur de leur seuil respectif $S_{1c}$ et $S_{2c}$. La détermination des seuils $S_{1t}$ , $S_{2t}$, $S_{1c}$ et $S_{2c}$ sera expliquée ci-après.

Selon que l'on connecte les moyens de visualisation 6 au signal numérique 25 ou 28, délivré respectivement par les premiers ou deuxièmes moyens de validation, on obtient l'image représentée à la figure 7E ou 7F. Sur la figure 7E apparaît nettement le défaut correspondant à un fil manquant en chaîne. Sur la figure 7F, ce défaut n'est pas détecté. Seuls des défauts correspondant à de petites irrégularités des fils de trame sont détectés. Il est possible à partir des deux images des figures 7E et 7F, de déterminer les frontières des défauts détectés et de faire apparaître lesdites frontières sur l'image initiale numérique de la figure 7A. Cette opération, par ailleurs connue de l'homme du métier sous le nom de segmentation, permet d'obtenir l'image représentée à la figure 7G.

Les moyens de traitement électronique 5 qui ont été décrits à titre d'exemple non limitatif, permettent d'effectuer en temps réel et simultanément l'acquisition de l'image initiale (étape a) et le filtrage de cette image (étape b). Cependant il est également possible de concevoir d'autres dispositifs de mise en oeuvre dans lesquels l'étape de filtrage est effectuée après que l'étape d'acquisition ait été réalisée. Dans ce cas, l'image initiale est par exemple sauvegardée, en vue de son filtrage, dans une mémoire vive.

De manière caractéristique, le procédé d'acquisition et de traitement d'image de l'invention comprend en outre avantageusement une étape supplémentaire dite d'apprentissage, qui est préalable à l'étape a) correspondant à l'acquisition de l'image initiale, et qui permet notamment de calculer automatiquement les vecteurs périodes $\vec{t}$ et $\vec{c}$.

Cette étape d'apprentissage est mise en oeuvre à l'aide de moyens de traitement électronique 7 appropriés, recevant en entrée le premier signal numérique 9, ainsi que le signal d'horloge 10. Les moyens de traitement électronique 7 vont à présent être décrits uniquement d'un point de vue fonctionnel, leur réalisation sur le plan structurel étant à la portée de l'homme du métier.

Dans un premier temps, par l'intermédiaire des moyens de traitement électronique 7, on acquiert une image dite d'apprentissage, à partir d'une portion de l'étoffe 1, ne comportant pas de défaut. Tous les niveaux de gris des points élémentaires constituant cette image d'apprentissage sont par exemple mémorisés dans une mémoire vive, et les moyens de traitement électronique 7 sont par exemple constitués par un microprocesseur, programmé en sorte de pouvoir calculer à partir de cette image d'apprentissage qui est mémorisée, les vecteurs période $\vec{t}$ et $\vec{c}$.

On a donné à titre d'exemple aux figures 9A et 9B les organigrammes de l'algorithme de calcul des vecteurs période $\vec{t}$ et $\vec{c}$.

Le contenu des différentes étapes des organigrammes des figures 9A et 9B est suffisamment explicite sur ces figures et ne sera donc pas répété dans la présente description. La réalisation de ces étapes permet le calcul automatique des coordonnées (Perx, Pery) du vecteur période $\vec{t}$ ou $\vec{c}$ selon la direction X ou Y choisie (figure 9A). Ce calcul est effectué à partir de plusieurs vecteurs dont les coordonnées (Retx, Rety) sont calculées à partir de valeurs prédéterminées (minret, Maxret, K). Pour illustrer le calcul de ces vecteurs dans la direction X, on a représenté schématiquement à la figure 8 une image d'apprentissage 36, de dimension DIMX et DIMY, sur laquelle apparaît une partie des vecteurs calculés. La réalisation des étapes des organigrammes des figures 9A et 9B revient à appliquer la succession d'étapes suivantes :

1) calculer pour chaque vecteur prédéterminé une image d'apprentissage filtrée, en appliquant les étapes b1) à b2) qui ont été précédemment décrites;
2) choisir pour vecteur période $\vec{t}$ ou $\vec{c}$, le vecteur prédéterminé pour lequel la somme des niveaux de gris de l'image d'apprentissage filtrée est minimale.

L'étape d'apprentissage consiste également à calculer les seuils $S_{1t}$, $S_{2t}$ à partir de l'image d'apprentissage filtrée associée au vecteur $\bar{t}$, et les seuils $S_{1c}$ et $S_{2c}$ à partir de l'image d'apprentissage filtrée associée au vecteur période $\bar{c}$. On a représenté à la figure 9C l'organigramme correspondant au calcul des seuils $S_{1t}$ et $S_{2t}$. A partir de l'image d'apprentissage filtrée associée au vecteur période $\vec{t}$, on calcule l'histogramme H(NG) des niveaux de gris de cette image. Sachant que cette image contient N points élémentaires, la fonction de répartition des niveaux de gris de cette image est :

$$F(S) = \sum_{NG=0}^{S} \frac{H(NG)}{N}$$

Le seuil $S_{1t}$ est choisi comme étant le plus petit seuil tel que F(S1) est supérieur ou égal à une valeur limite $1 - \varepsilon$ . Dans un exemple précis de réalisation, $\varepsilon$ valait 0,025.

Le seuil $S_{2t}$ est quant à lui déterminé à partir du seuil $S_{1t}$, par l'équation suivante :

$$S_{2t} = 3\sqrt{F(S_{1t})(1-F(S_{1t}))n} + n(1-F(S_{1t}))$$

n représente le nombre de points élémentaires contenus dans la fenêtre élémentaire qui sera utilisée à l'étape c) du procédé de l'invention et qui correspond à la validation des défauts dans l'image en noir et blanc obtenue à l'étape b3).

Le calcul automatique des seuils $S_{2t}$ et $S_{2c}$ est réalisé de la même manière par un organigramme identique, à partir de l'image d'apprentissage filtrée associée au vecteur $\vec{c}$.

Le procédé d'acquisition et de traitement de l'invention qui a été décrit n'est pas limité à la détection de défauts de fabrication dans une étoffe de tissu, mais peut être utilisé pour détecter toute irrégularité dans la structure d'un article plan à structure périodique. Cet article plan peut par exemple être également un article de textile tricoté.

## Revendications

1. Procédé d'acquisition et de traitement de l'image d'un article plan à structure périodique dans au moins une direction, du type étoffe de textile tissée, en vue de la détection de défauts de fabrication , consistant :

a) à acquérir une image initiale de la surface de l'article, laquelle image se caractérise au moins par un vecteur période $\vec{t}$ (respectivement $\vec{c}$),

b) et simultanément ou successivement à filtrer l'image initiale en vue d'obtenir une image filtrée en noir et blanc ,

b1) en calculant pour chaque point élément P de l'image initiale, un niveau de gris NG'(P) qui est proportionnel à la valeur absolue de la différence des niveaux de gris dans l'image initiale du point élémentaire P et de son homologue A (respectivement D) par la translation de vecteur période $\vec{t}$ (respectivement $\vec{c}$),

b2) en affectant au point élémentaire P, le niveau de gris calculé à l'étape précédente,

b3) et en rendant binaire le niveau de gris de chaque point élémentaire P par comparaison avec un premier seuil $S_{1t}$ (respectivement $S_{1c}$) prédéterminé ,

caractérisé en ce qu'il comprend une étape préalable dite d'apprentissage qui comporte la succession d'étapes suivantes :

- acquérir une image numérique dite d'apprentissage d'une partie de la surface de l'article qui est dépourvue de défauts,
- appliquer les étapes b1) à b2) précitées sur l'image d'apprentissage ,
- calculer l'histogramme des niveaux de gris de l'image d'apprentissage filtrée de vecteur période $\vec{t}$ (respectivement $\vec{c}$) ,
- calculer automatiquement le premier seuil $S_{1t}$ (respectivement $S_{1c}$) à partir de l'histogramme des niveaux de gris de l'image d'apprentissage filtrée.

**2.** Procédé selon la revendication 1 caractérisé en ce qu'il consiste, à partir de l'histogramme de l'image d'apprentissage filtrée, à calculer la fonction de répartition F (S) des niveaux de gris, et à en déduire le premier seuil $S_{1t}$ (respectivement $S_{1C}$).

**3.** Procédé selon la revendication 2 caractérisé en ce que le premier seuil $S_{1t}$ (respectivement $S_{1\ C}$) est calculé automatiquement à partir d'un coefficient ε prédéterminé inférieur à 1, et correspond au plus petit seuil S tel que F(S) est supérieur ou égal à (1 - ε).

**4.** Procédé selon la revendication 3 caractérisé en ce que ε vaut O,O25.

**5.** Procédé selon la revendication 1 caractérisé en ce qu'il comprend après l'étape b) , une étape supplémentaire c) consistant à transformer l'image filtrée en noir et blanc obtenue , d'une part en calculant pour chaque point élémentaire P , la somme des niveaux de gris des points élémentaires situés dans une fenêtre élémentaire qui est de forme et de dimension prédéterminées, et qui contient le point élémentaire P, et d'autre part en affectant la valeur binaire O ou 1 au niveau de gris de chaque point élémentaire P, en fonction du résultat de la comparaison de ladite somme avec un deuxième seuil $S_{2t}$ (respectivement $S_{2c}$) prédéterminé.

**6.** Procédé selon les revendications 2 et 5 caractérisé en ce que la fenêtre élémentaire utilisée à l'étape c) contenant n points élémentaires, le procédé consiste en outre, au cours de l'étape d'apprentissage, à déterminer automatiquement le deuxième seuil $S_{2t}$ (respectivement $S_{2c}$) en calculant l'expression :

$$3\sqrt{F(S_{1t})(1-F(S_{1t}))n} + n(1-F(S_{1t}))$$

$$(\text{respectivement } 3\sqrt{F(S_{1c})(1-F(S_{1c}))n} + n\,(1-F(S_{1c})))$$

**7.** Procédé selon l'une des revendications 1 à 6 caractérisé en ce qu'il consiste en outre à effectuer à l'étape b1), le calcul d'un niveau de gris NG'' (P) qui est proportionnel à la valeur absolue de la différence des niveaux de gris, dans l'image initiale, du point élémentaire P et du point élémentaire C (respectivement B) dont P est l'homologue par la translation de vecteur période $\vec{t}$ (respectivement $\vec{c}$), et en ce qu'il comprend une étape supplémentaire b'1) préalable à l'étape b2) consistant à déterminer un niveau de gris NG (P) qui est fonction des niveaux de gris NG'(P) et NG''(P).

**8.** Procédé selon la revendication 7 caractérisé en ce que le niveau de gris NG (P) est proportionnel à la plus petite valeur des deux niveaux de gris NG'(P) et NG''(P).

**9.** Procédé selon la revendication 7 caractérisé en ce que le niveau de gris NG(P) est proportionnel à la somme des

deux niveaux de gris NG'(P) et NG"(P).

**10.** Procédé selon l'une des revendications 1, 7, 8 ou 9 caractérisé en ce que l'étape d'apprentissage consiste en outre à déterminer automatiquement le vecteur période $\vec{t}$ (respectivement $\vec{c}$) de l'image d'apprentissage, parmi plusieurs vecteurs prédéterminés, en effectuant la succession d'étapes suivantes :

1) calculer pour chaque vecteur prédéterminé une image d'apprentissage filtrée, en appliquant les étapes b1) à b2) pour chaque point élémentaire de l'image d'apprentissage,
2) choisir pour vecteur période le vecteur prédéterminé pour lequel la somme des niveaux de gris de l'image d'apprentissage filtrée est minimale.

**Claims**

**1.** A process for acquiring and dealing with the image of a flat article with a repeat structure in at least one direction, of the woven textile material type, with a view to detecting manufacturing faults, consisting in:

a) acquiring an initial image of the surface of the article, this image being characterised at least by a repeat vector $\vec{t}$ (or $\vec{c}$).

b) and, simultaneously or successively, filtering the initial image with a view to obtaining a black and white filtered image,

b1) by calculating, for each elementary point P of the initial image, at the level of grey NG'(P) which is proportional to the absolute value of the difference in the levels of grey in the initial image of the elementary point P and its counterpart A (or D) by linear movement of the repeat vector $\vec{t}$ (or $\vec{c}$)

b2) by allocating to the elementary point P the level of grey calculated in the previous stage;

b3) and by making binary the level of grey of each elementary point P in comparison with an initial predetermined threshold $S_{1t}$ (or $S_{1c}$),
characterised by the fact that it includes a prior stage, called the learning stage, which contains the following stages:

- acquiring a digital image, called the learning image, of a part of the surface of the article which is free from faults;
- applying the above mentioned stages b1) to b2) to the learning image;
- calculating the histogram of the levels of grey of the repeat vector $\vec{t}$ (or $\vec{c}$) filtered learning image;
- automatically calculating the first threshold $S_{1t}$ (or $S_{1c}$) from the histogram of the levels of grey of the filtered learning image.

**2.** A process in accordance with Claim 1 characterised by the fact that it consists, from the histogram of the filtered learning image, in calculating the distribution function F (S) of the levels of grey and deducing therefrom the first threshold $S_{1t}$ (or $S_{1c}$).

**3.** A process in accordance with Claim 2 characterised by the fact that the first threshold $S_{1t}$ (or $S_{1c}$) is automatically calculated from a predetermined coefficient $\varepsilon$ and corresponds to the smallest threshold S so that F(S) is above or equal to (1 - $\varepsilon$).

**4.** A process in accordance with Claim 3 characterised by the fact that $\varepsilon$ is worth 0.025.

**5.** A process in accordance with Claim 1 characterised by the fact that after stage b) it has an extra stage c) consisting in converting the black and white filtered image obtained, on the one hand by calculating for each elementary point P the sum of the levels of grey of the elementary points located in an elementary window which is of a predetermined form and size and contains the elementary point P and, on the other hand, by allocating the binary value 0 or 1 to the level of grey of each elementary point P, as a function of the result of the comparison of the aforesaid sum with a second predetermined threshold $S_{2t}$ (or $S_{2c}$).

**6.** A process in accordance with Claims 2 and 5 characterised by the fact that, with the elementary window used at

stage c) containing n elementary points, the process consists in addition, during the learning stage, in automatically determining the second threshold $S_{2t}$ (or $S_{2c}$) by calculating the expression:

$$3\sqrt{F(S_{1t})(1 - F(S_{1t}))n} + n(1 - F(S_{1t}))$$

$$\text{or } 3\sqrt{F(S_{1c})(1 - F(S_{1c}))n} + n(1 - F(S_{1c}))$$

**7.** A process in accordance with one of the Claims 1 to 6 characterised by the fact that it consists in addition to carrying out stage b1), calculation of a level of grey NG" (P) which is proportional to the absolute value of the difference in the levels of grey, in the initial image, of the elementary point P and of the elementary point C (or B), of which P is the counterpart through the linear movement of repeat vector $\vec{t}$ (or $\vec{c}$), and by the fact that it includes an extra stage b'1) prior to stage b2) consisting in determining a level of grey NG (P) which is a function of the levels of grey NG'(P) and NG"(P)

**8.** A process in accordance with Claim 7 characterised by the fact that the level of grey NG (P) is proportional to the smallest figure for the two levels of grey NG'(P) and NG"(P).

**9.** A process in accordance with Claim 7 characterised by the fact that the level of grey NG(P) is proportional to the sum of the two levels of grey NG'(P) and NG"(P).

**10.** A process in accordance with Claims 1, 7, 8 or 9 characterised by the fact that the learning stage consists in addition in automatically determining the repeat vector $\vec{t}$ (or $\vec{c}$) of the learning image from among several predetermined vectors, by carrying out the sequence of the following stages:

1) calculating, for each predetermined vector, a filtered learning image by applying stages b1) to b2) for each elementary point of the learning image;
2) choosing for the repeat vector the predetermined vector for which the sum of the levels of grey of the filtered learning image is minimal.

**Patentansprüche**

**1.** Verfahren zur Erfassung und Verarbeitung des Bilds eines flachen Artikels mit in mindestens einer Richtung periodisch wiederkehrender Struktur nach Art eines gewebten Textilstoffs im Hinblick auf die Erkennung von Produktionsfehlern, das darin besteht,

a) ein Ursprungsbild der Oberfläche des Artikels zu erfassen, welches Bild zumindest durch einen Periodenvektor $\vec{t}$ (bzw. $\vec{c}$) gekennzeichnet ist,
b) und gleichzeitig oder nacheinander das Ursprungsbild im Hinblick auf den Erhalt eines gefilterten Schwarzweißbilds zu filtern,

b1) indem für jeden elementaren Punkt P des Ursprungsbilds eine Graustufe NG'(P) berechnet wird, die proportional zum absoluten Wert des Unterschieds der Graustufen in dem Ursprungsbild des elementaren Punkts P und seiner Entsprechung A (bzw.) D durch Translation des Periodenvektors $\vec{t}$ (bzw. $\vec{c}$) ist,
b2) indem dem elementaren Punkt P die im vorangehenden Schritt berechnete Graustufe zugewiesen wird,
b3) und indem die Graustufe jedes elementaren Punkts P durch Vergleich mit einem ersten, vorbestimmten Schwellenwert $S_{1t}$ (bzw. $S_{1c}$) binär gemacht wird,
dadurch gekennzeichnet, dass es einen vorausgehenden Schritt umfasst, der Lernschritt genannt wird und die Folge der folgenden Schritte umfasst:

- Erfassung eines digitalen, sog. Lernbilds eines fehlerfreien Teils der Oberfläche des Artikels,
- Anwendung der vorgenannten Schritte b1) bis b2) auf das Lernbild,
- Berechnung des Histogramms der Graustufen des gefilterten Lernbilds mit Periodenvektor $\vec{t}$ (bzw. $\vec{c}$),
- automatische Berechnung des ersten Schwellenwerts $S_{1t}$ (bzw. $S_{1c}$) auf der Grundlage des Histogramms der Graustufen des gefilterten Lernbilds.

**2.** Verfahren nach Anspruch 1, dadurch gekennzeichnet, dass es darin besteht, ausgehend von dem Histogramm des gefilterten Lernbilds die Verteilungsfunktion F (S) der Graustufen zu berechnen und daraus den ersten Schwellen-

wert $S_{1t}$ (bzw. $S_{1c}$) abzuleiten.

3. Verfahren nach Anspruch 2, dadurch gekennzeichnet, dass der erste Schwellenwert $S_{1t}$ (bzw. $S_{1c}$) automatisch ausgehend von einem vorbestimmten Koeffizienten ε, der kleiner ist als 1, berechnet wird und dem kleinsten Schwellenwert S entspricht, so dass F(S) größer oder gleich (1-ε) ist.

4. Verfahren nach Anspruch 3, dadurch gekennzeichnet, dass ε gleich 0,025 ist.

5. Verfahren nach Anspruch 1, dadurch gekennzeichnet, dass es nach dem Schritt b) einen zusätzlichen Schritt c) aufweist, der darin besteht, das erhaltene gefilterte Schwarzweißbild umzuwandeln, und zwar zum einen durch Berechnung der Summe der Graustufen der elementaren Punkte, die in einem elementaren Fenster gelegen sind, dessen Form und Abmessungen vorbestimmt sind und das den elementaren Punkt P enthält, für jeden elementaren Punkt P und zum anderen, indem der Graustufe jedes elementaren Punkts P der Binärwert 0 oder 1 zugewiesen wird, und zwar abhängig von dem Ergebnis des Vergleichs der genannten Summe mit einem zweiten, vorbestimmten Schwellenwert $S_{2t}$ (bzw. $S_{2c}$).

6. Verfahren nach den Ansprüchen 2 und 5, dadurch gekennzeichnet, dass das Verfahren, nachdem das in Schritt c) verwendete elementare Fenster n elementare Punkte enthält, während der Lernphase ferner darin besteht, automatisch den zweiten Schwellenwert $S_{2t}$ (bzw. $S_{2c}$) zu bestimmen, indem folgender Ausdruck berechnet wird:

$$3\sqrt{F(S_{1t})\,(1-F(S_{1t}))n} + n(1-F(S_{1t}))$$

$$(\text{beziehungsweise } 3\sqrt{F(S_{1c})\,(1-F(S_{1c}))\,n} + n\,(1-F(S_{1c})))$$

7. Verfahren nach einem der Ansprüche 1 bis 6, dadurch gekennzeichnet, dass es außerdem darin besteht, in Schritt b1) die Berechnung einer Graustufe NG'' (P) durchzuführen, die proportional zum absoluten Wert des Unterschieds der Graustufen des elementaren Punkts P und des elementaren Punkts C (bzw. B) in dem Ursprungsbild ist, dem P durch die Translation des Periodenvektors $\vec{t}$ (bzw. $\vec{c}$) entspricht, sowie dadurch, dass es einen zusätzlichen Schritt b'1) umfasst, der dem Schritt b2) vorausgeht und darin besteht, eine Graustufe NG (P) zu bestimmen, die abhängig von den Graustufen NG'(P) und NG''(P) ist.

8. Verfahren nach Anspruch 7, dadurch gekennzeichnet, dass die Graustufe NG (P) proportional zum kleinsten Wert der beiden Graustufen NG'(P) und NG''(P) ist.

9. Verfahren nach Anspruch 7, dadurch gekennzeichnet, dass die Graustufe NG (P) proportional zur Summe der beiden Graustufen NG'(P) und NG''(P) ist.

10. Verfahren nach einem der Ansprüche 1, 7, 8 oder 9, dadurch gekennzeichnet, dass der Lernschritt ferner in der automatischen Bestimmung des Periodenvektors $\vec{t}$ (bzw. $\vec{c}$) des Lernbilds aus mehreren vorbestimmten Vektoren besteht, indem die Folge folgender Schritte ausgeführt wird:

   1) Berechnung eines gefilterten Lernbilds für jeden vorbestimmten Vektor, durch Anwendung der Schritte b1) bis b2) für jeden elementaren Punkt des Lernbilds,
   2) Wahl des vorbestimmten Vektors als Periodenvektor, bei dem die Summe der Graustufen des gefilterten Lernbilds die kleinste ist.

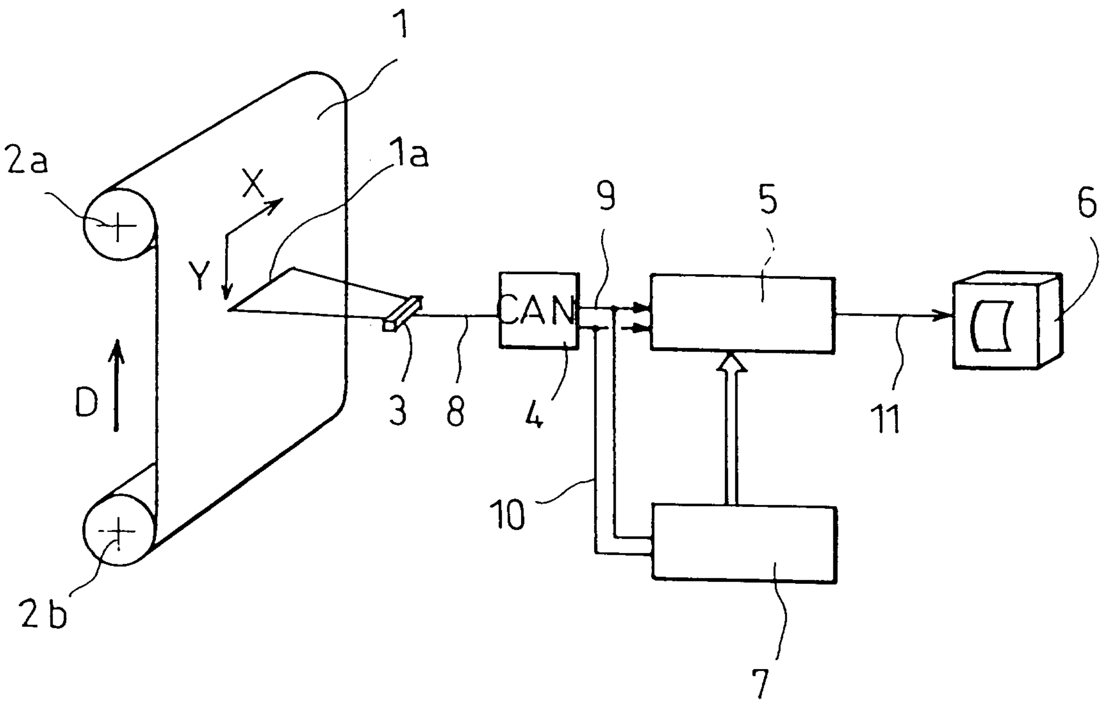


FIG_1

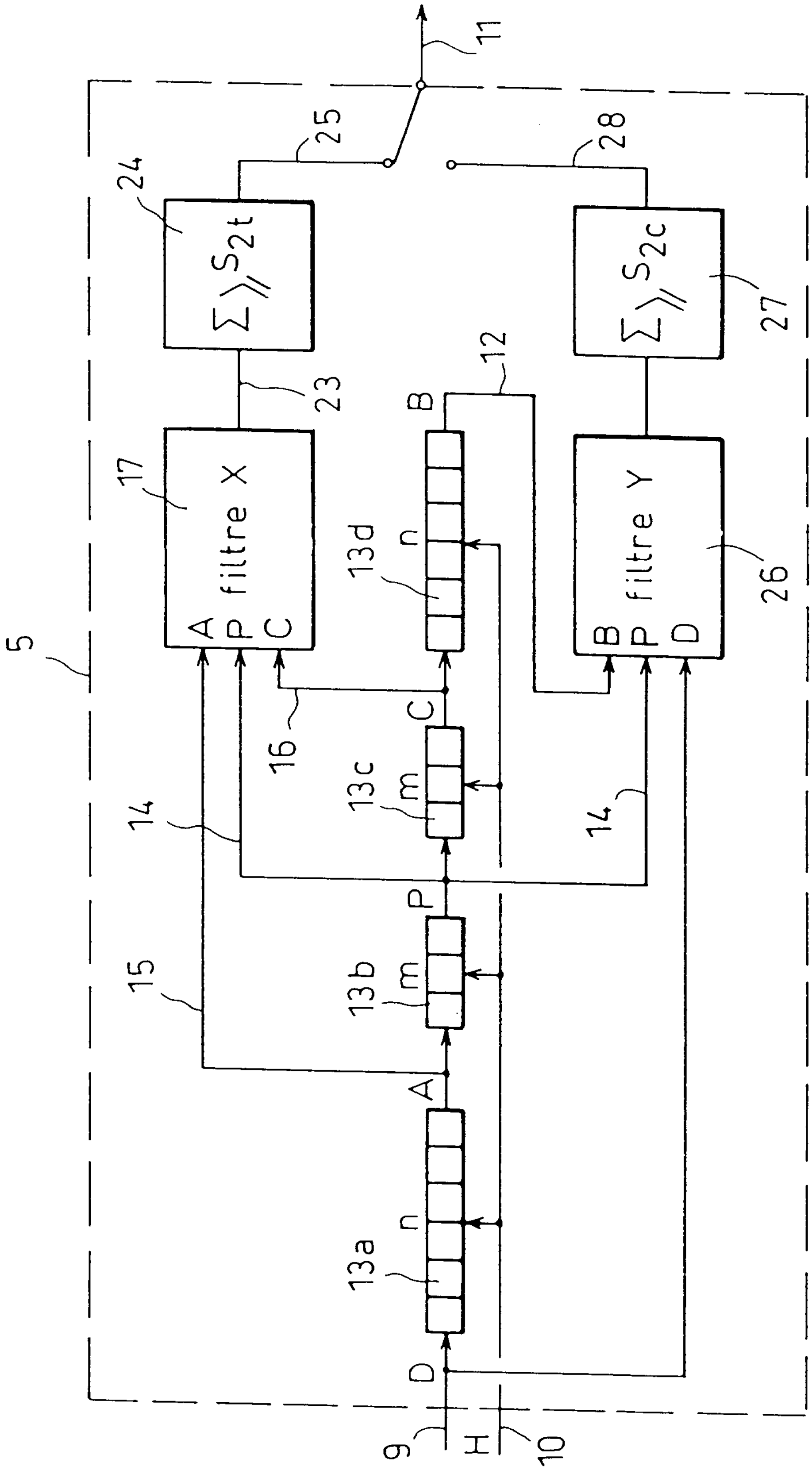
5
9
H
10
D
13a
n
A
13b
m
15
P
14
13c
m
16
C
13d
n
B
12
filtre X
A
P
C
17
23
24
Σ ≥ S2t
25
11
28
27
Σ ≥ S2c
26
filtre Y
B
P
D
14

FIG_2

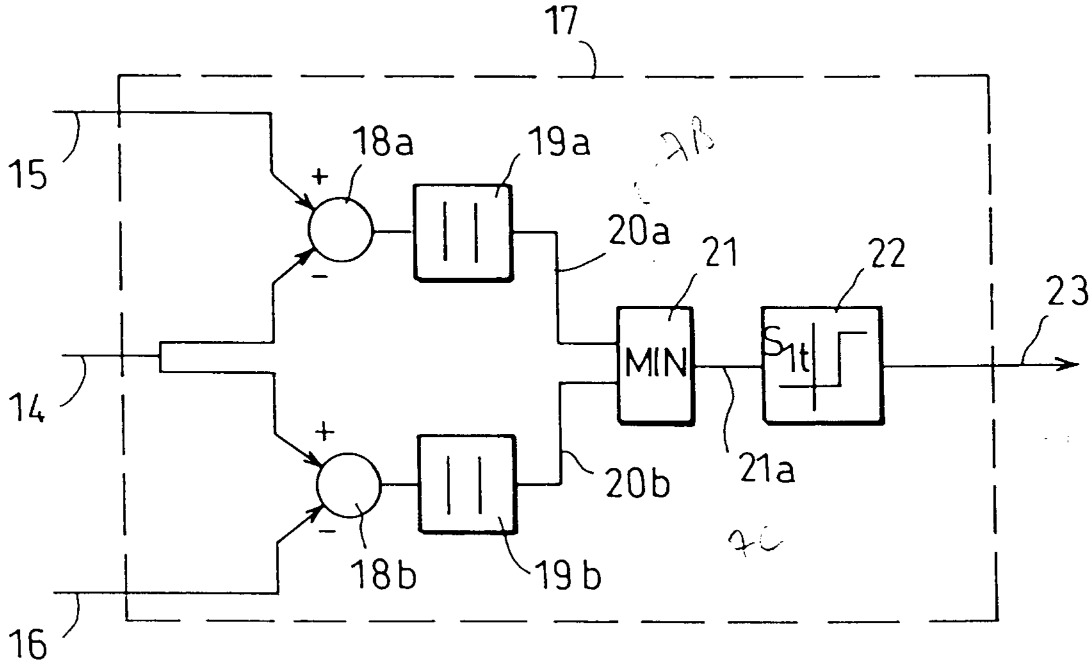
17
15
18a
+
-
19a
20a
21
MIN
22
S1t
23
14
21a
+
20b
-
18b
19 b
16

FIG_3A

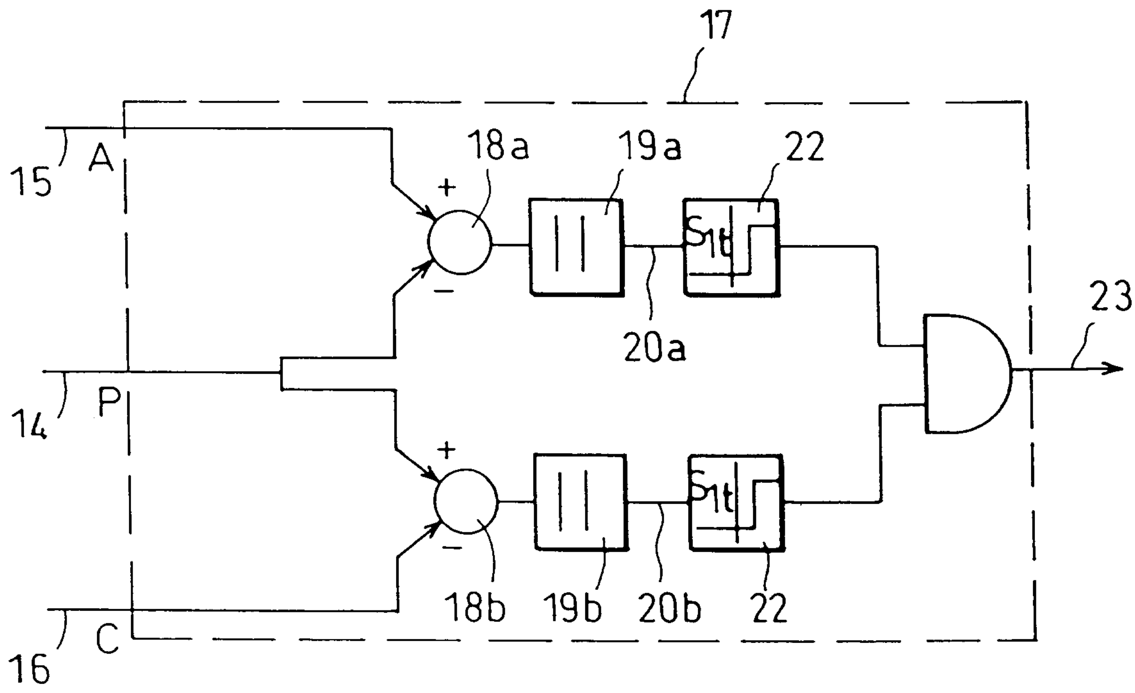
17
15
A
18a
+
19a
22
S1t
-
20a
23
14
P
+
S1t
-
18b
19b
20b
22
16
C

FIG_3 B

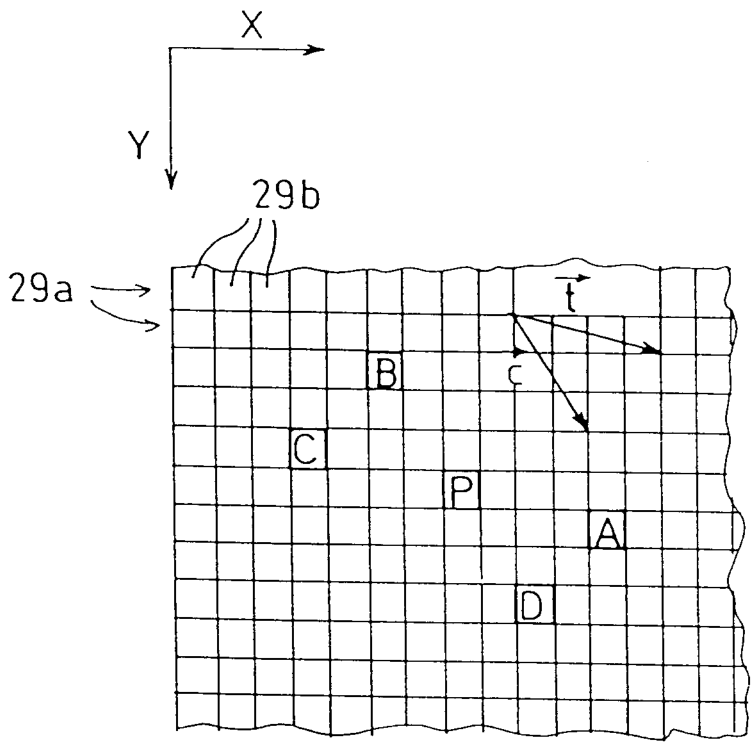


FIG_4

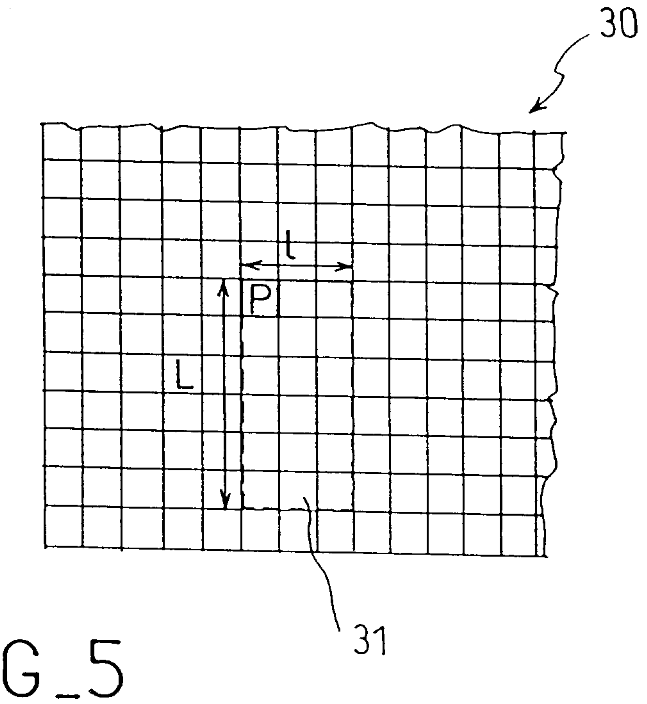


FIG_5

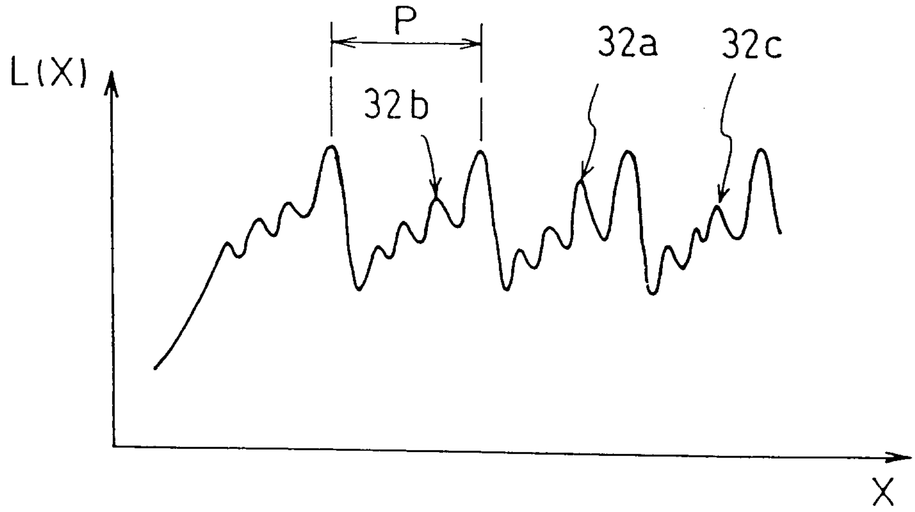


FIG_6A

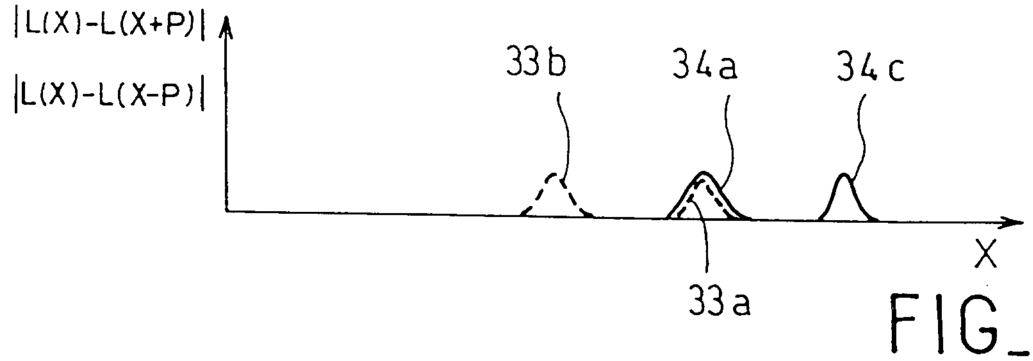


FIG_6B

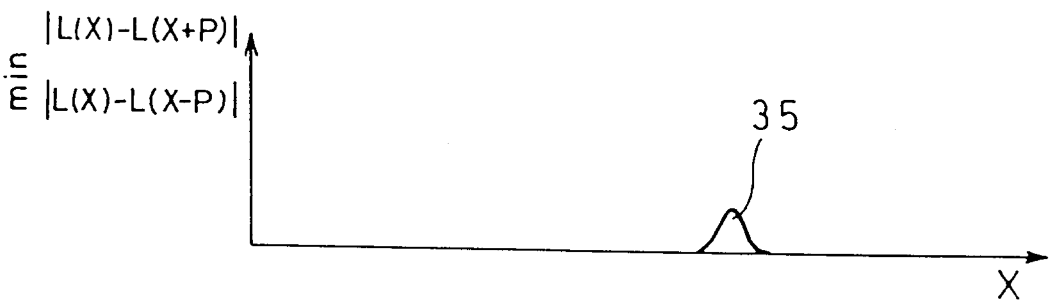


FIG_6C

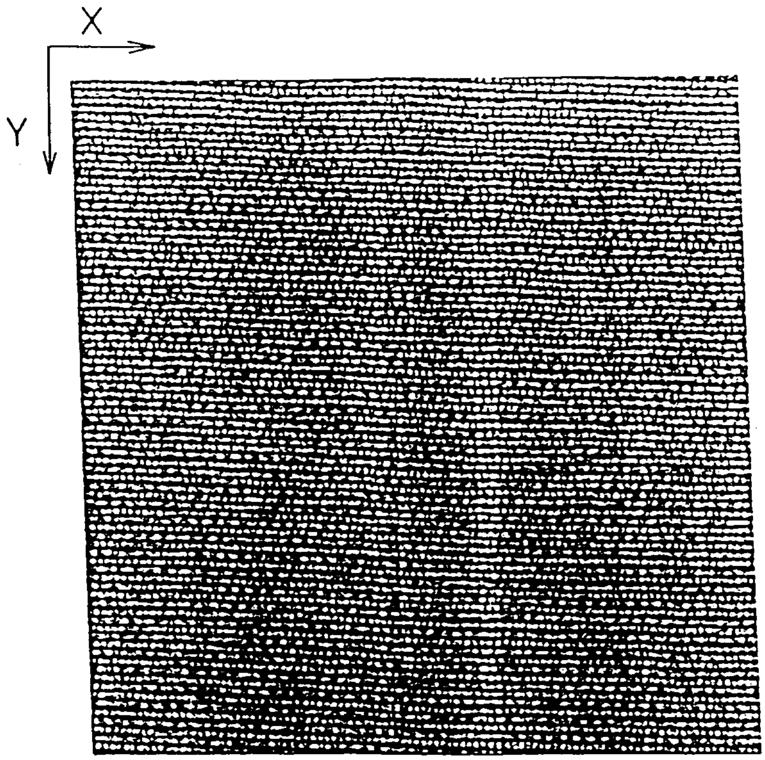


FIG_7A

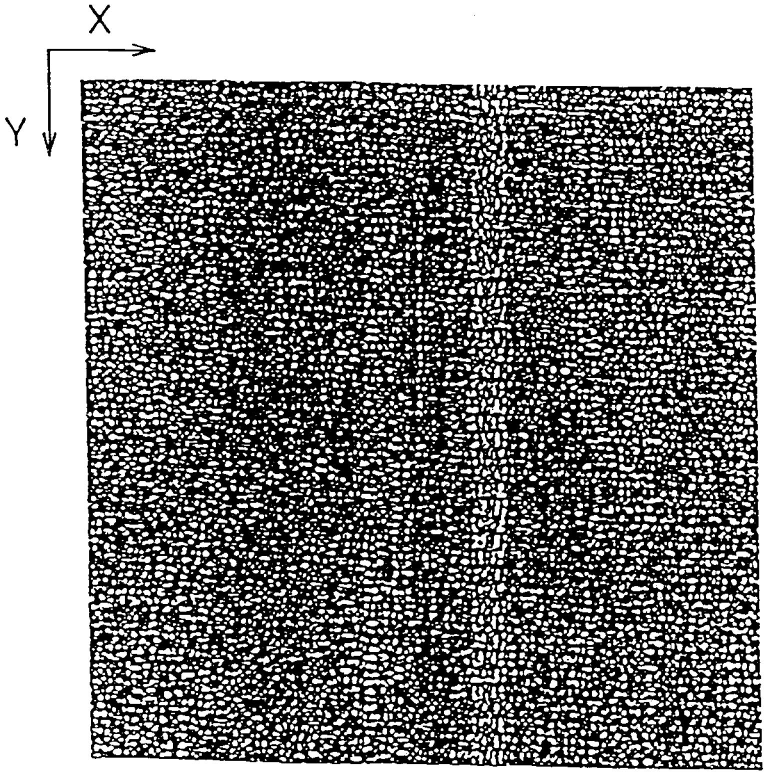


FIG_7B

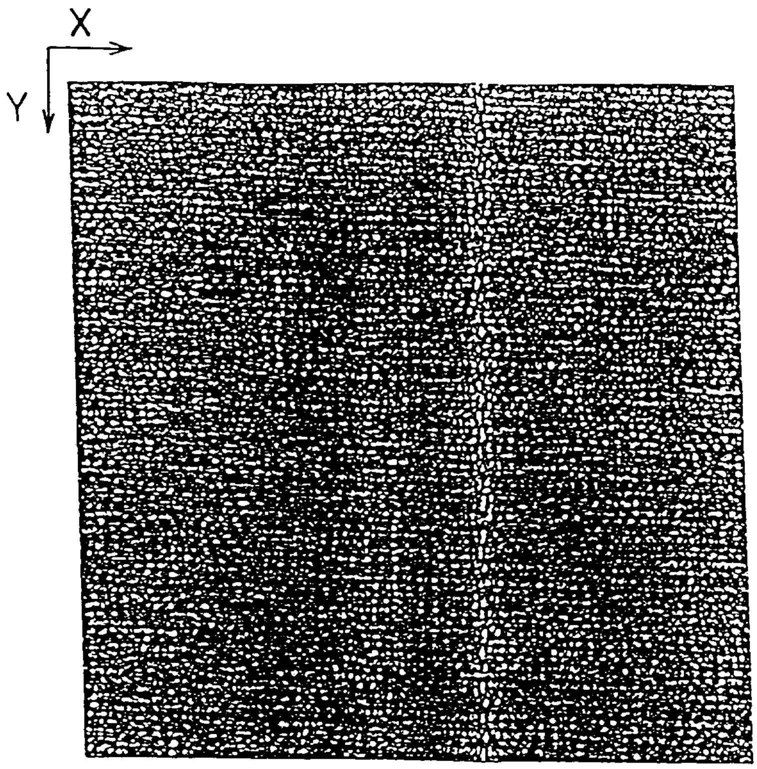


FIG_7C

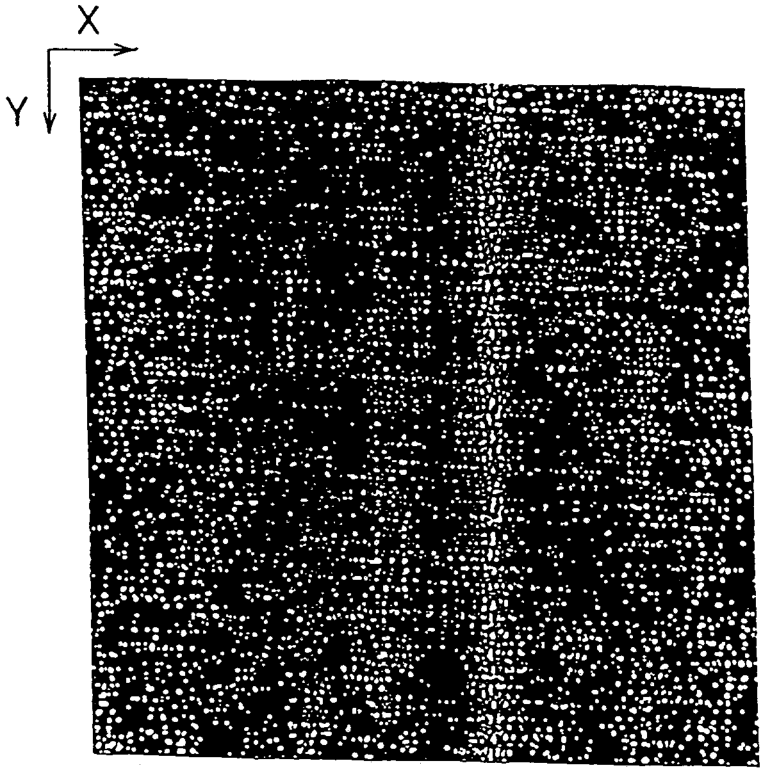


FIG_7D

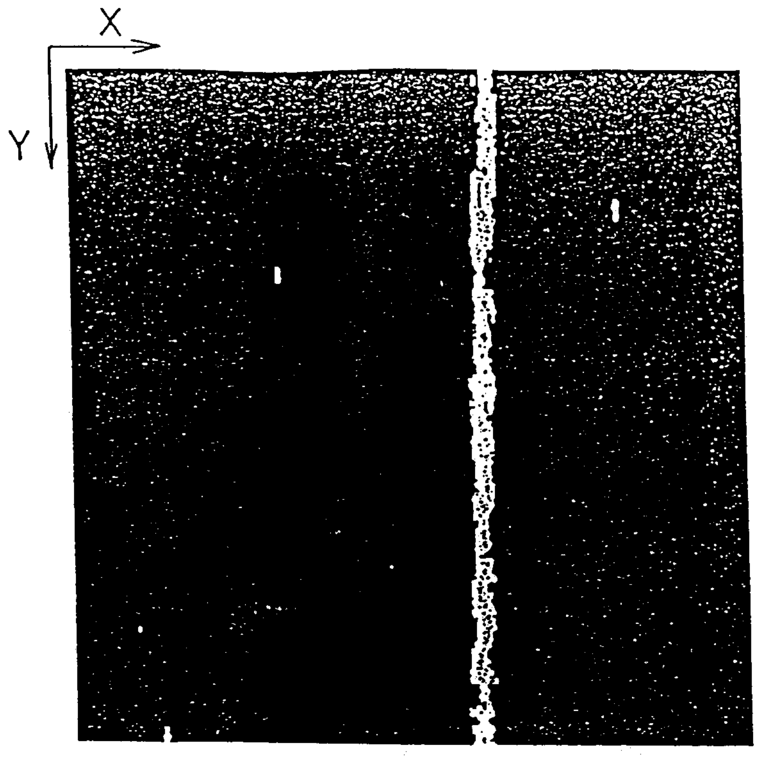


FIG_7E

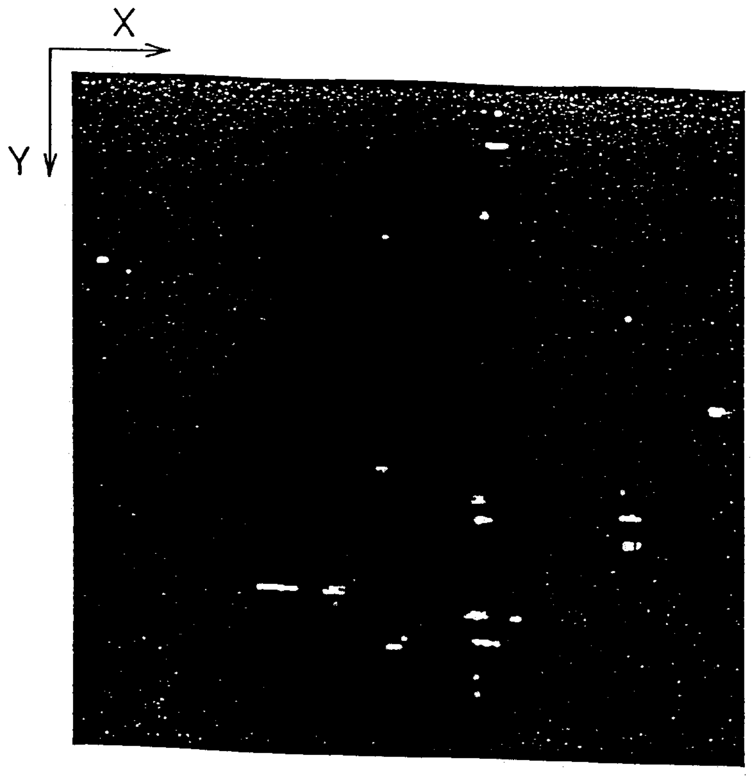


FIG_7F

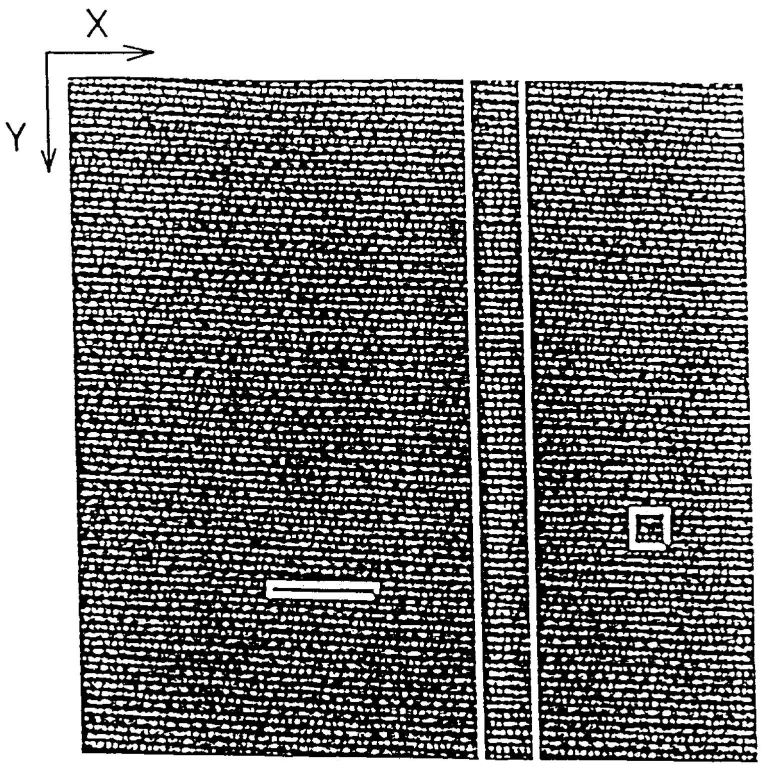


FIG_7G

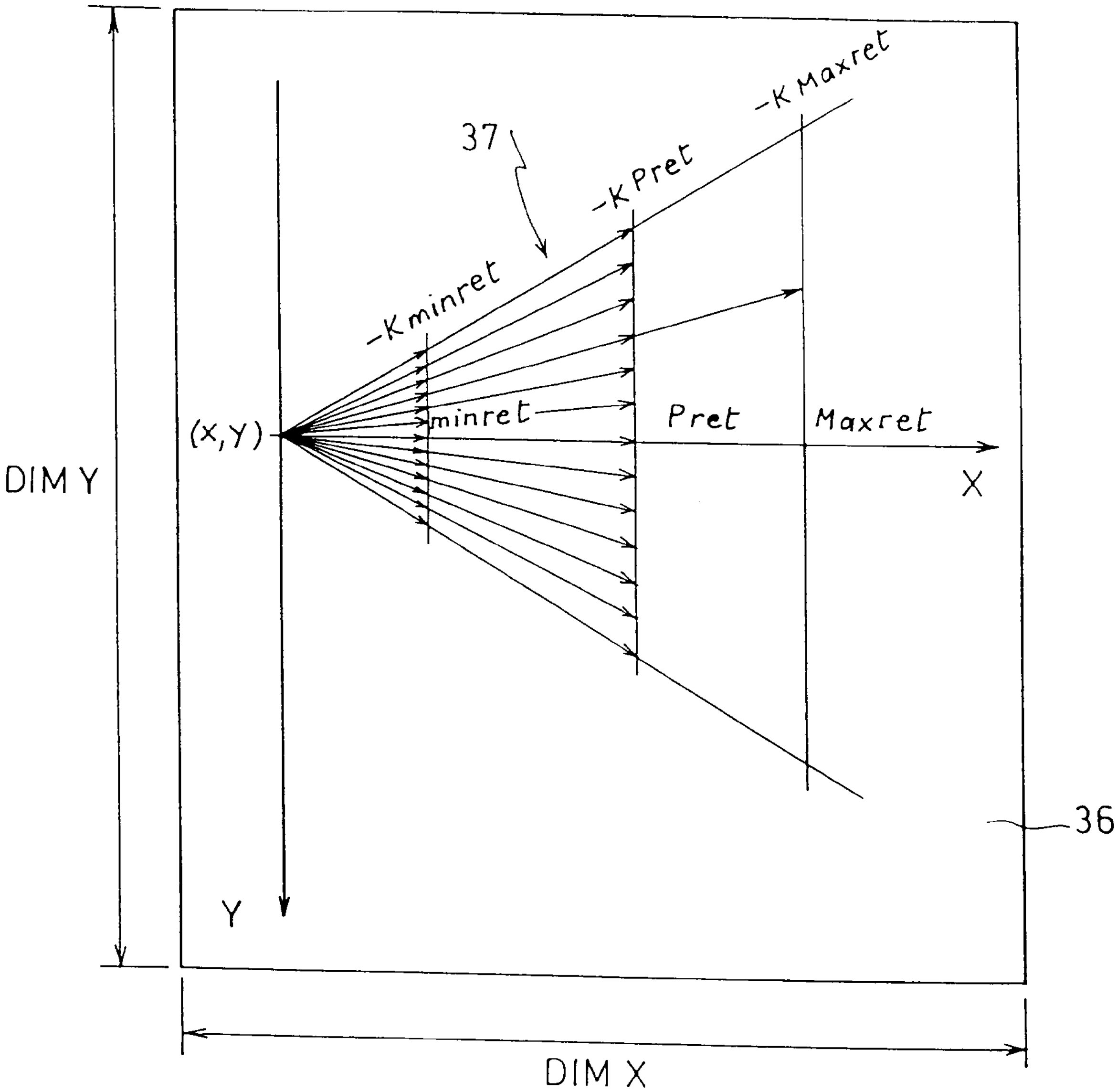
37
-K Maxret
-K Pret
-K minret
(X,Y)
minret
Pret
Maxret
X
DIM Y
Y
36
DIM X

FIG_8

FIG_9A

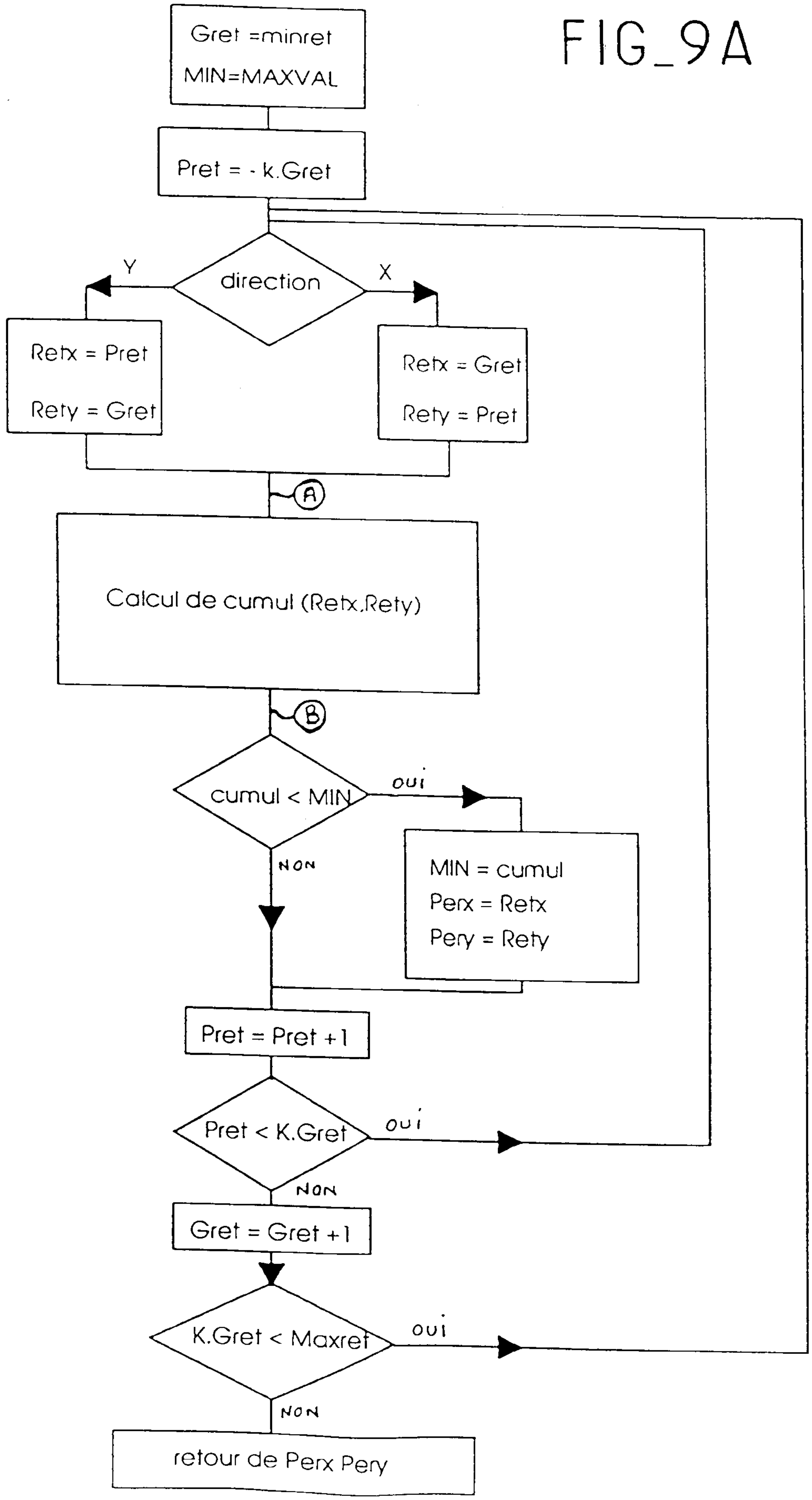
Gret =minret
MIN=MAXVAL
Pret = - k.Gret
direction
Y
X
Retx = Pret
Rety = Gret
Retx = Gret
Rety = Pret
A
Calcul de cumul (Retx,Rety)
B
cumul < MIN
oui
NON
MIN = cumul
Perx = Retx
Pery = Rety
Pret = Pret +1
Pret < K.Gret
oui
NON
Gret = Gret +1
K.Gret < Maxret
oui
NON
retour de Perx Pery

FIG_9B

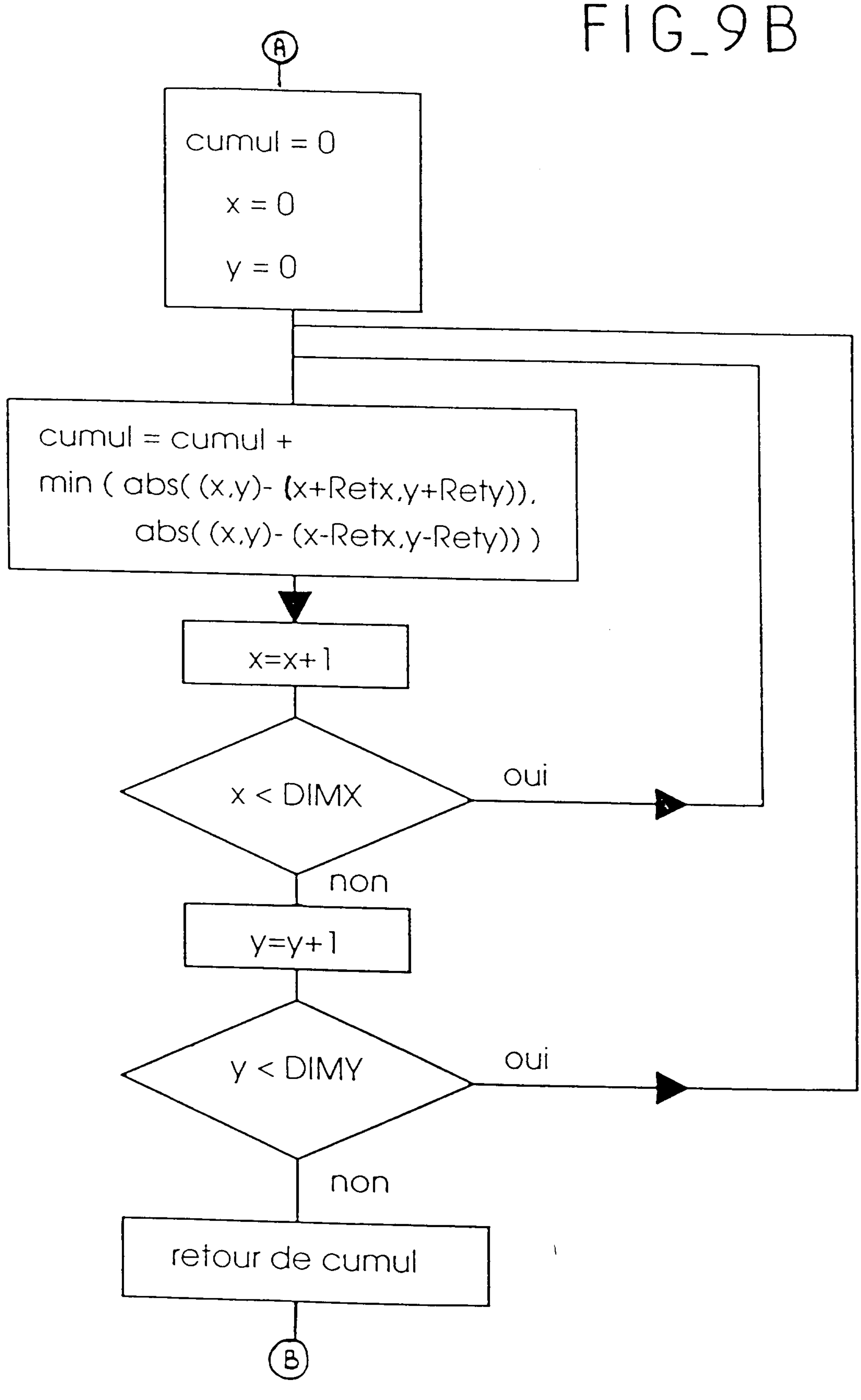
A
cumul = 0
x = 0
y = 0
cumul = cumul +
min ( abs( (x,y)- (x+Retx,y+Rety)),
abs( (x,y)- (x-Retx,y-Rety)) )
x=x+1
x < DIMX
oui
non
y=y+1
y < DIMY
oui
non
retour de cumul
B

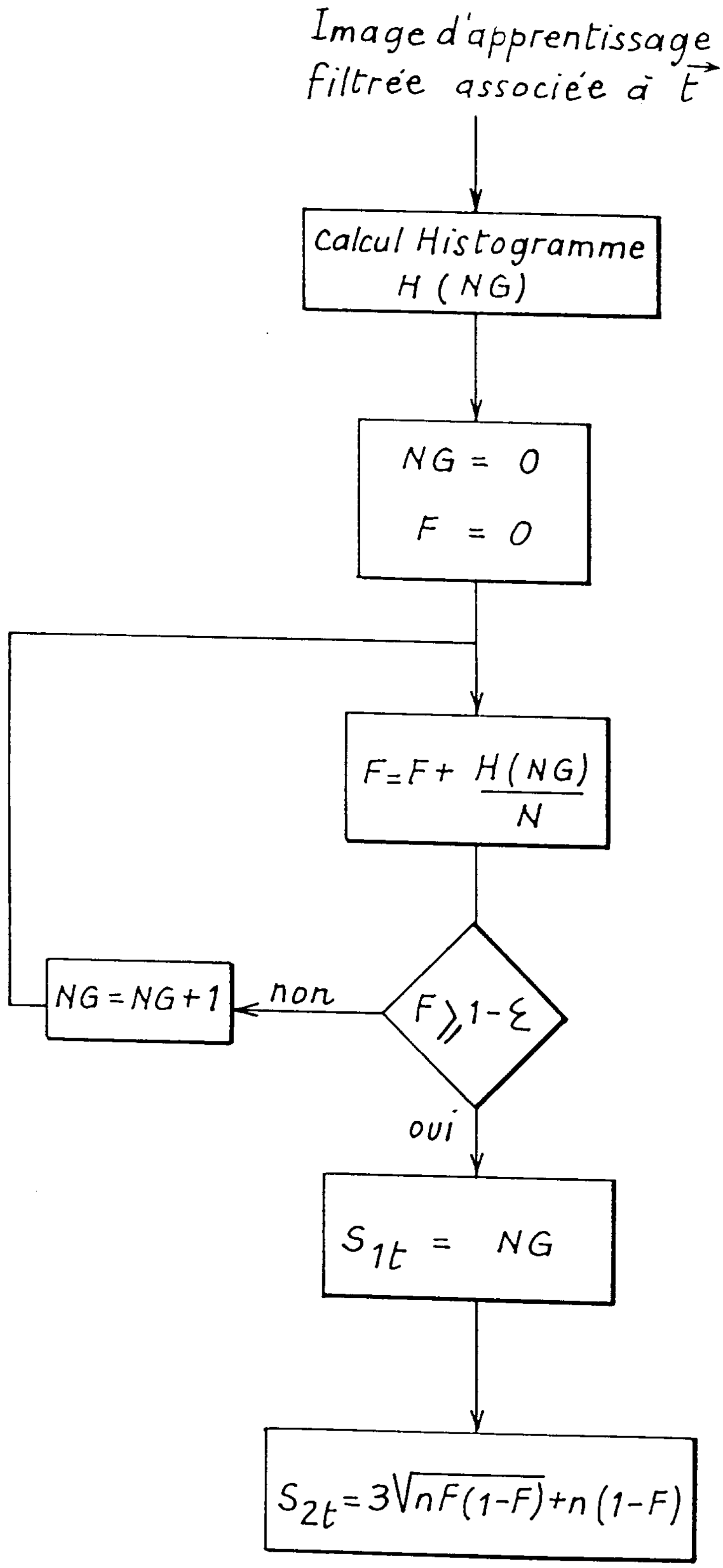
Image d'apprentissage filtrée associée à $\vec{t}$
Calcul Histogramme H (NG)
NG = 0
F = 0
F=F+ $\frac{H(NG)}{N}$
F ≥ 1-ε
non
NG=NG+1
oui
$S_{1t}$ = NG
$S_{2t}=3\sqrt{nF(1-F)}+n(1-F)$

FIG_9C